# EUROPEAN PATENT APPLICATION

(11) **EP 2 674 845 A1**
(43) Date of publication of application: **18.12.2013**
(21) Application number: 12171966.0
(22) Date of filing: 14.06.2012
(51) Int. Cl.: G06F 3/048

(54) **User interaction via a touch screen**

(71) Applicant: ICT Automatisering N.V., 2993 LL Barendrecht (NL)
(72) Inventor: Wissink, Jorg Petrus Aloysius, 5091 EK Middelbeers (NL); de Ru, Patrick, 4175 CG Haaften (NL)
(74) Representative: Nederlandsch Octrooibureau

(57) **Abstract**

A system for user interaction via a touch screen comprises a first touch unit (1) for detecting a first touch event associated with a first interactive area of a touch screen, wherein the first interactive area is associated with an operation type. A data display unit (3) is arranged for displaying at least part of a dataset in a second interactive area of the touch screen. A second touch unit (2) is arranged for detecting a touch move event associated with the second interactive area. An associating unit (4) is arranged for associating the operation type with the touch move event if the touch move event was detected while a touch of the first interactive area associated with the first touch event is still ongoing. An operation unit (5) is arranged for determining an operation based on the operation type and the touch move event.

## Description

### FIELD OF THE INVENTION

The invention relates to user interaction via a touch screen. The invention further relates to a medical image viewer.

### BACKGROUND OF THE INVENTION

In healthcare, images of patients are acquired using image acquisition devices. Such images include X-ray images, computed tomography images, MRI images, and other kinds of medical images.

After acquisition, the images are reviewed by a radiologist or other healthcare professional. To this end, image viewing workstations are known to provide functionality of displaying an image. The display of an image is known to be dependent on user configurable parameters. Such parameters may include a zoom level, panning parameters, contrast/brightness, window width/window level, and other parameters. Moreover, in a three-dimensional or higher dimensional dataset, there may be user configurable parameters that define what portion of the image dataset is shown on the display. For example, a slice may be selected for viewing. Moreover, in some cases, it may be possible to perform processing operations on an image, for example to enhance details using an edge enhancement operation, or other operations. Such operations may involve a user configurable parameter. Moreover, functionality may be provided to draw objects on top of the image dataset, using the mouse pointer or a touch screen interface.

In other fields of image viewing, similar user-configurable parameters may be supported to enable a user to influence the display and/or the contents of the image dataset. For example, photographic images and video tracks may be displayed, wherein the display is dependent on the user configurable parameters. Moreover, image processing operations may be performed on such images using user configurable image processing parameters.

Other kinds of datasets, such as text documents, may also be displayed based on user configurable parameters, such as scrolling level and/or zoom level. Also the contents of the document may be changed by a user.

### SUMMARY OF THE INVENTION

It is an object of the invention to provide improved user interaction via a touch screen.

In a first aspect, the invention provides a system comprising:
a first touch unit for detecting a first touch event associated with a first interactive area of a touch screen, wherein the first interactive area is associated with an operation type;
a data display unit for displaying at least part of a dataset in a second interactive area of the touch screen;
a second touch unit for detecting a touch move event associated with the second interactive area; and
an associating unit for associating the operation type with the touch move event if the touch move event was detected while a touch of the first interactive area associated with the first touch event is still ongoing.

This way, the user can select the type of operation that the user wishes to perform by touching the first interactive area of the touch screen. While continuing to touch the first interactive area, the user may cause a touch move event associated with the second interactive area of the touch screen. The selected operation type is then associated with the touch move event. This may make the behavior of the system more predictable, because the selection of the operation type is valid as long as the touch of the first interactive area continues. After that, an inadvertent touch move event would not cause the operation type to be associated with such inadvertent touch move event. This way, a user can operate the system with improved confidence. Alternatively or additionally, the described solution may improve the efficiency of user interactions, because the user may more quickly select a type of operation and perform the desired adjustment.

The system may comprise an operation unit for determining an operation based on the operation type and the touch move event. The data generated by the touch move event may be combined with the operation type to determine a specific operation in detail. Such an operation may include, besides the operation type, a parameter derived from the touch move event. The data generated by the touch move event, which may be taken into account by the operation unit, may include at least one of: a start point and/or an end point of the touch, a direction of travel, a distance traveled, and a distance traveled in a predetermined direction.

The operation type may entail an adjustment of a parameter, wherein the adjustment is dependent on the touch move event.

The second touch unit may be arranged for determining a travel distance associated with the touch move event. Moreover, the operation unit may be arranged for determining the adjustment of the parameter in dependence on the travel distance. This is a suitable way of determining the adjustment.

The second touch unit may be arranged for determining a direction of travel associated with the touch move event. Moreover, the operation unit may be arranged for determining the adjustment of the parameter in dependence on the direction of travel. Using the direction of travel may be used as an alternative way of determining the parameter adjustment.

The first interactive area may be associated with a plurality of operation types. Moreover, the operation unit may be arranged for determining at least a first adjustment of a first one of the plurality of operation types and a second adjustment of a second one of the plurality of operation types, in dependence on the travel distance and the direction of travel. This makes it possible to adjust a plurality of parameters with a single moving touch gesture.

The data display unit may be arranged for displaying said at least part of the dataset in dependence on at least one viewing parameter, and wherein the operation type entails an adjustment of the viewing parameter, wherein the adjustment is dependent on the touch move event.

The operation type may entail an operation on at least part of the dataset, wherein the operation has an operation parameter associated therewith, and wherein the parameter is dependent on the touch move event.

The first touch unit may be arranged for detecting a touch end event associated with an end of the touch of the first interactive area associated with the first touch event. Moreover, the associating unit may be arranged for not associating the operation type with a touch move event associated with the second interactive area and detected after the touch end event.

The dataset may comprise an image dataset, for example a medical image dataset.

Another aspect of the invention provides a device comprising a touch screen and the system set forth. For example, the device is a mobile terminal such as a tablet or mobile phone.

Another aspect of the invention provides a medical image viewer comprising the system set forth..

Another aspect of the invention provides a method of user interaction via a touch screen, comprising
displaying at least part of a dataset in a second interactive area of a touch screen;
detecting a first touch event associated with a first interactive area of the touch screen, wherein the first interactive area is associated with an operation type;
detecting a touch move event associated with the second interactive area, while the first touch event is ongoing; and
associating the operation type with the touch move event.

Another aspect of the invention provides a computer program product comprising instructions for causing a processor system to perform the method set forth or to implement the system set forth.

The person skilled in the art will understand that the features described above may be combined in any way deemed useful. Moreover, modifications and variations described in respect of the system may likewise be applied to the method and to the computer program product, and modifications and variations described in respect of the method may likewise be applied to the system and to the computer program product.

### BRIEF DESCRIPTION OF THE DRAWINGS

In the following, aspects of the invention will be elucidated by means of examples, with reference to the drawings. The drawings are diagrammatic and may not be drawn to scale.
Fig. 1 is a block diagram of a system for user interaction via a touch screen.
Fig. 2 is a flowchart of a method of user interaction via a touch screen.
Fig. 3 is a sketch of a touch screen touched by a user.

### DETAILED DESCRIPTION OF EMBODIMENTS

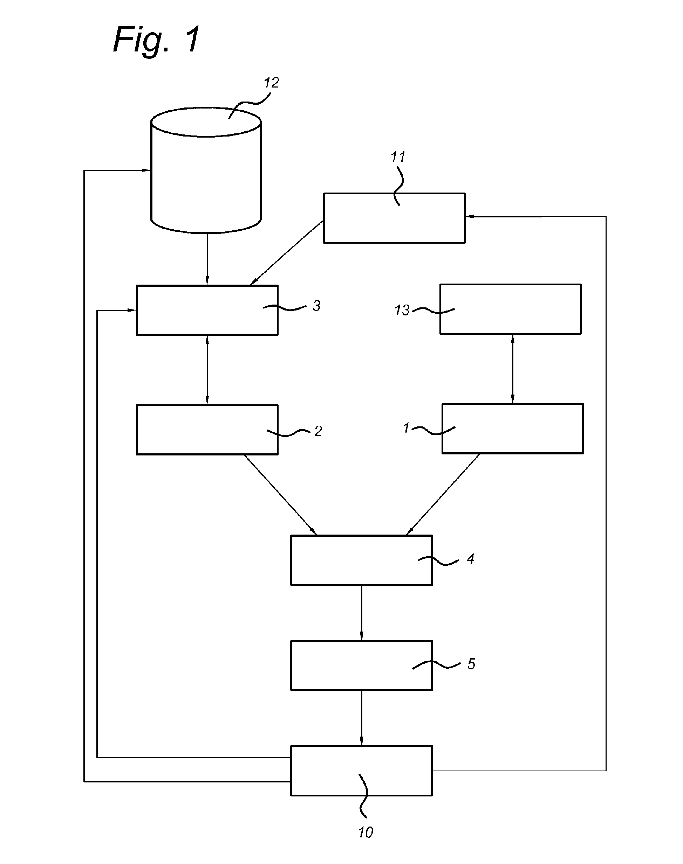

Fig. 1 illustrates aspects of a system for user interaction via a touch screen. The system may be implemented on a device having a touch screen. The system may also be implemented in a computing device that is operatively connected to a separate touch screen device. The system may also be implemented at least partly by means of software. Alternatively, the system may be implemented by means of dedicated electronic circuitry. The system may comprise a communications interface for providing a connection to a network or to another device. Such a connection may be used, for example, to retrieve a dataset from a server, and/or to communicate annotations, remarks, or other information to the server and/or to other users. The system may have a processor and/or a storage device for storing and executing the software, and/or for storing, processing, and displaying the dataset.

The touch screen, possibly in conjunction with appropriate software module, may be arranged for generating events in response to a user touching the touch screen. Information included in such an event may include a location where the touch screen was touched, a duration of the touch, and a possible movement of the touch, in case the user moves her finger over the screen or otherwise generates a moving touch gesture.

The display may be divided logically into certain interactive areas, wherein different control mechanisms are associated with different interactive areas.

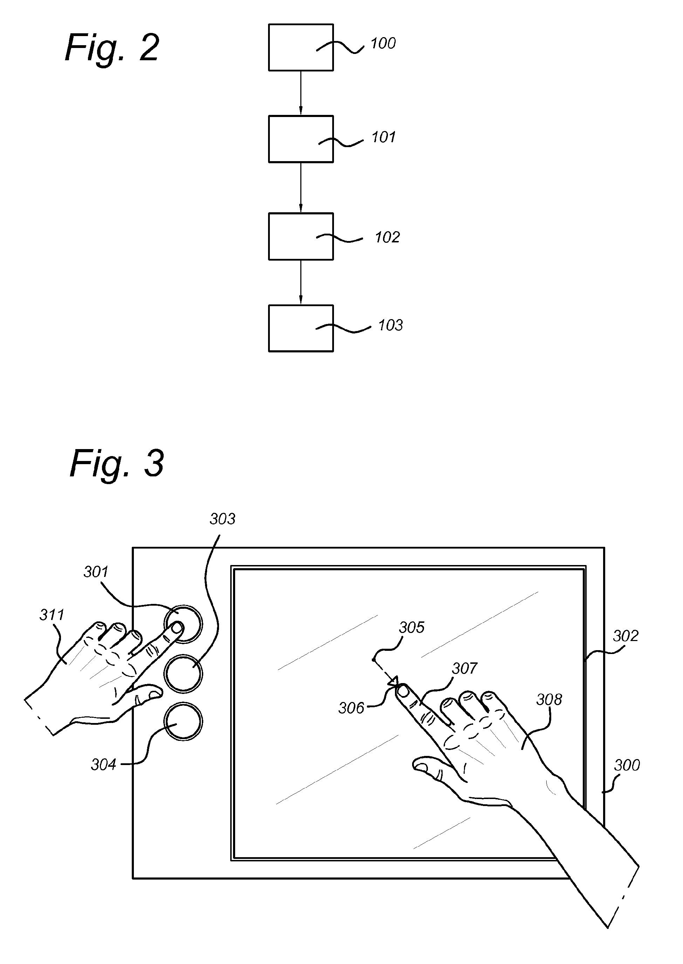

Fig. 3 shows an example layout of a touch screen 300 that is touched in two places, namely by a finger 310 of a user's left hand 311 and by a finger 307 of a user's right hand 308. In the drawing of the touch screen 300, four first interactive areas 301, 302, 303, and 304 are illustrated.

With reference to both Fig. 1 and Fig. 3, the system may comprise a first touch unit 1 operatively coupled to the touch screen and arranged for detecting a first touch event associated with a first interactive area 301 of the touch screen 300, wherein the first interactive area 301 is associated with an operation type. The operation type can be any one of many different kinds of operation types, including setting the window width, window level, and other kinds of operation types. Illustratively shown in Fig. 3 are more interactive areas 303, 304. These interactive areas may be associated with different operation types. This way, the user can select a different operation type by touching the appropriate interactive area 301, 303, or 304. The touch of the first interactive area activates an operating mode of the system, in such a manner that any touch move event in the second interactive area is associated with the operation type of the first interactive area 301. This operating mode ends when the touch of the first interactive area ends.

The system may comprise a control display unit 13 arranged for displaying an indication of the first interactive area 301 on the touch screen 300. For example, the control display unit 13 may be arranged for displaying an icon or a text label at the position of the first interactive area 301. The control display unit 13 may be operatively coupled to the first touch unit 1 in such a way as to visually indicate when the first interactive area is being touched. For example, the color of the first interactive area 301 may be changed when the first interactive area 301 is touched. Other ways of making such a visual indication may also be provided. Moreover, when the first interactive area 301 is no longer touched, the visual indication in the first interactive area 301 may return to the same state as before the touch event occurred.

It is possible that the first interactive area 301 is configurable. For example, the location and size of the first interactive area 301 may be changed. For example, these may be adjusted to the vertical or horizontal orientation of the touch screen device. The control display unit 13 may be arranged for displaying the indication of the first interactive area 301 in a semi-transparent way, to enable the user to see some of the dataset 'behind' the indication of the interactive area 301. In case there are a plurality of first interactive areas 301, 303, 304, associated with different operation types, these first interactive areas 301, 303, 304 may be distinguished by arranging the control display unit 13 to display a different indication on each first interactive area 301, 303, 304.

The system may comprise a data display unit 3 operatively coupled to the touch screen and arranged for displaying at least part of a dataset in the second interactive area 302 of the touch screen. For example, the system may offer functionality to enable a user to select a dataset from a list or database of datasets. The system may also offer functionality to retrieve the dataset from a remote system via the network. These functionalities are not described in further detail herein. The skilled person will be able to provide the functionality to select, retrieve, and display a dataset in the second interactive area 302. At least part of the dataset may be displayed in the second interactive area 302. For example, the user may browse or scroll through the dataset. Such browsing or scrolling may be performed using the interactive techniques described herein, or by means of conventional interactive techniques. The system may be arranged for displaying the dataset before the first touch unit detects the first touch event.

The system may comprise a second touch unit 2 operatively coupled to the touch screen and arranged for detecting a touch move event associated with the second interactive area, while a touch of the first interactive area associated with the first touch event is ongoing.

The system may comprise an associating unit 4 arranged for associating the operation type with the touch move event. The associating unit 4 may be operatively coupled to the first touch unit 1 and the second touch unit 2. Moreover, the associating unit 4 may be arranged for verifying whether the touch of the first interactive area 301 associated with the first touch event is still ongoing at the time of detecting the touch move event. The associating unit 4 may be arranged for associating the operation type with the touch move event only in case the touch of the first interactive area 301 associated with the first touch event is still ongoing, i.e., the user has not lifted his finger from the touch screen. Optionally the associating unit 4 may check whether the user's finger has left the first interactive area, and associate the operation type with the touch move event only in case the touch of the first interactive area 301 associated with the first touch event has not left the first interactive area. However, this is not a limitation.

The system may comprise an operation unit 5 arranged for determining an operation based on the operation type and the touch move event. Data of the touch move event may be used to specify an operation. Such data may include direction of touch and/or distance of touch. Alternatively, such data may include movement distance in X direction and movement distance in Y direction, for example. The data may be used to select a particular operation subtype within the operation type, for example.

The operation type may entail an adjustment of a parameter, wherein the adjustment is dependent on the touch move event. The distance of travel may be used to determine the adjustment of the parameter. For example, a linear relationship may be used between distance of travel and amount of adjustment. The distance of travel in a particular direction, such as an X direction or an Y direction (e.g. horizontal or vertical direction) may be used alternatively.

The second touch unit 2 may be arranged for determining a travel distance associated with the touch move event. The travel distance may be determined as a distance from a first point 305 where the user first touched the second interactive area 302 to a second point 306 where the user currently touches the touch screen 302, or where the user stops touching the touch screen 302.

The operation unit 5 may be arranged for determining the adjustment of the parameter in dependence on the travel distance. The operation unit 5 may be arranged for updating the adjustment of the parameter continuously while the touch of the touch move event is still ongoing, whenever a further movement of the touched area is detected. Alternatively, the operation unit 5 may be arranged for determining the adjustment of the parameter only once, when the user stops touching the touch screen.

The second touch unit 2 may be arranged for determining a direction of travel associated with the touch move event. The operation unit 5 may be arranged for determining the adjustment of the parameter in dependence on the direction of travel.

The first interactive area may be associated with a plurality of operation types. The operation unit 5 may be arranged for determining at least a first adjustment of a first one of the plurality of operation types and a second adjustment of a second one of the plurality of operation types, in dependence on the travel distance and the direction of travel. More operation types may be supported, for example by means of more travel directions.

The data display unit 3 may be arranged for displaying said at least part of the dataset in dependence on at least one viewing parameter, and wherein the operation type entails an adjustment of the viewing parameter, wherein the adjustment is dependent on the touch move event.

The operation type may entail an operation on at least part of the dataset, wherein the operation has an operation parameter associated therewith, and wherein the parameter is dependent on the touch move event.

The first touch unit 1 may be arranged for detecting a touch end event associated with an end of the touch of the first interactive area associated with the first touch event. The associating unit 4 is arranged for not associating the operation type with any touch move event associated with the second interactive area and detected after the touch end event.

The dataset may comprise an image dataset. For example, the system may be implemented as a user interaction module of an image viewer. The system may also be a medical image viewer for displaying a medical image dataset in the second interactive area.

The system may comprise a parameter unit 11 to store the parameter value and forward the parameter value to the data display unit 3. The system may comprise an operation execution unit 10 for performing the operation as determined by the operation unit 5. The operation execution unit 10 may be arranged for performing the operation in response to any (further) move associated with the touch of the touch move event. Alternatively, the operation execution unit 10 may be arranged for performing the operation in response to a touch end event associated with an end of the touch of the touch move event. Other operation execution frequencies are also possible.

Operation types supported by the first interactive area 301, 303, 304 may include any one or more of the following: contrast/brightness setting, window width and window level setting, series navigation: to select an image of a series of images, panning: changing the field of view, zooming: changing the zoom level.

Aspects of the system may be configurable. For example, the type of icon shown in a first interactive area 301 may be changed. The kind of operation may be selectable, for example a different image processing operation may be associated with the first interactive area 301. The first interactive area 301 may also be user-configurable to become a switch. In such a case, when the first interactive area 301 is configured to be a switch, the user may select the operation type by touching the first interactive area 301, and may release the touch of the first interactive area 301. After that, the user may generate the touch move event in the second interactive area, and the associating unit associates the operation type with the touch move event. When the user deselects the operation type by touching the first interactive area 301 again, the associating unit does no longer associate the operation type with any subsequent touch move event. When the user configures the first interactive area 301 to operate in 'smart mode', the functionality described in respect of Fig. 1 is performed again.

A device, such as a tablet computer or another device comprising a touch screen may be provided with the system set forth herein.

Fig. 2 illustrates aspects of a method of user interaction via a touch screen. The method may comprise step 100 of displaying at least part of a dataset in a second interactive area of a touch screen. The method may further comprise step 101 of detecting a first touch event associated with a first interactive area of the touch screen, wherein the first interactive area is associated with an operation type. The method may further comprise step 102 of detecting a touch move event associated with the second interactive area, while the touch of the first interactive area that caused the first touch event is ongoing. The method may further comprise step 103 of associating the operation type with the touch move event.

The method may be extended and/or modified based on the functionality described herein with respect to the system. The method may be implemented completely or partly in software.

Some or all aspects of the invention may be suitable for being implemented in form of software, in particular a computer program product. Such computer program product may comprise a storage media on which the software is stored. Such a storage media may comprise, for example, an optical disc, magnetic disk, or flash memory. Also, the computer program may be represented by a signal, such as an optic signal or an electro-magnetic signal, carried by a transmission medium such as an optic fiber cable or the air. The computer program may partly or entirely have the form of source code, object code, or pseudo code, suitable for being executed by a computer system. For example, the code may be directly executable by one or more processors. Alternatively, the code may be interpreted by an interpreter that is executed by one or more processors. It will be understood that portions of the systems described herein may be implemented in form of software. Moreover, the method steps described herein may be implemented partially or completely in software. The software may be organized by means of subroutines. The subroutines may be combined to form a standalone executable program. Alternatively, the subroutines may be organized as a dynamically linkable library. A main program executable file may be provided that uses the subroutines from the dynamically linkable library. Each of the processing steps and/or system components described herein may be represented by executable code, be it in a dynamically linked library or in an executable file. Some, or all, of the functionality may be implemented as part of an operating system, some functionality may be implemented in a dynamically linked library, and some functionality may be implemented as an application program file.

The examples and embodiments described herein serve to illustrate rather than limit the invention. The person skilled in the art will be able to design alternative embodiments without departing from the scope of the claims. Reference signs placed in parentheses in the claims shall not be interpreted to limit the scope of the claims. Items described as separate entities in the claims or the description may be implemented as a single hardware or software item combining the features of the items described.

## Claims

1. A system for user interaction via a touch screen, comprising
a first touch unit (1) for detecting a first touch event associated with a first interactive area of a touch screen, wherein the first interactive area is associated with an operation type;
a data display unit (3) for displaying at least part of a dataset in a second interactive area of the touch screen;
a second touch unit (2) for detecting a touch move event associated with the second interactive area; and
an associating unit (4) for associating the operation type with the touch move event if the touch move event was detected while a touch of the first interactive area associated with the first touch event is still ongoing.

2. The system according to claim 1, comprising
an operation unit (5) for determining an operation based on the operation type and the touch move event.

3. The system according to claim 1 or 2, wherein the operation type entails an adjustment of a parameter, wherein the adjustment is dependent on the touch move event.

4. The system according to claim 3,
wherein the second touch unit (2) is arranged for determining a travel distance associated with the touch move event; and
wherein the operation unit (5) is arranged for determining the adjustment of the parameter in dependence on the travel distance.

5. The system according to claim 3 or 4,
wherein the second touch unit (2) is arranged for determining a direction of travel associated with the touch move event; and
wherein the operation unit (5) is arranged for determining the adjustment of the parameter in dependence on the direction of travel.

6. The system according to claim 5,
wherein the first interactive area is associated with a plurality of operation types;
and
wherein the operation unit (5) is arranged for determining at least a first adjustment of a first one of the plurality of operation types and a second adjustment of a second one of the plurality of operation types, in dependence on the travel distance and the direction of travel.

7. The system according to claim 1, wherein the data display unit (3) is arranged for displaying said at least part of the dataset in dependence on at least one viewing parameter, and wherein the operation type entails an adjustment of the viewing parameter, wherein the adjustment is dependent on the touch move event.

8. The system according to claim 1, wherein the operation type entails an operation on at least part of the dataset, wherein the operation has an operation parameter associated therewith, and wherein the parameter is dependent on the touch move event.

9. The system according to claim 1,
wherein the first touch unit (1) is arranged for detecting a touch end event associated with an end of the touch of the first interactive area associated with the first touch event; and
wherein the associating unit (4) is arranged for not associating the operation type with a touch move event associated with the second interactive area and detected after the touch end event.

10. The system according to claim l, wherein the dataset comprises an image dataset.

11. The system according to claim 9, wherein the image dataset is a medical image dataset.

12. A device comprising a touch screen and the system according to claim 1.

13. A medical image viewer comprising the system according to claim 1.

14. A method of user interaction via a touch screen, comprising
displaying (100) at least part of a dataset in a second interactive area of a touch screen;
detecting (101) a first touch event associated with a first interactive area of the touch screen, wherein the first interactive area is associated with an operation type;
detecting (102) a touch move event associated with the second interactive area, while the first touch event is ongoing; and
associating (103) the operation type with the touch move event.

15. A computer program product comprising instructions for causing a processor system to perform the method according to claim 14.
